**Europäisches Patentamt**

**European Patent Office**

**Office européen des brevets**

(19)

(11) Publication number : **0 509 162 B1**

## (12) EUROPEAN PATENT SPECIFICATION

(45) Date of publication of patent specification :
**08.06.94 Bulletin 94/23**

(51) Int. Cl.[5] : **C10L 1/02**

(21) Application number : **91303339.5**

(22) Date of filing : **16.04.91**

(54) **Conversion of light hydrocarbons to ether rich gasolines.**

(43) Date of publication of application :
**21.10.92 Bulletin 92/43**

(45) Publication of the grant of the patent :
**08.06.94 Bulletin 94/23**

(84) Designated Contracting States :
**BE DE FR GB IT NL**

(56) References cited :
**EP-A- 0 172 040**
**US-A- 4 830 635**
**US-A- 4 925 455**
**US-A- 5 013 329**

(72) Inventor : **Bell, Weldon Kay**
**428, Burd Street**
**Pennington, New Jersey 08534 (US)**
Inventor : **Haag, Werner Otto**
**38, Pine Knoll Drive**
**Lawrenceville, New Jersey 08648 (US)**
Inventor : **Harandi, Mohsen Nadimi**
**12 Catbird Court**
**Lawrenceville, New Jersey 08648 (US)**
Inventor : **Owen, Hartley**
**5, Riverview Terrace**
**Belle Mead, New Jersey 08502 (US)**

(74) Representative : **Colmer, Stephen Gary et al**
**European Office of Patent Counsel**
**Mobil House**
**54-60 Victoria Street**
**London SW1E 6QB (GB)**

(73) Proprietor : **MOBIL OIL CORPORATION (a**
**New York corporation)**
**3225 Gallows Road**
**Fairfax, Virginia 22037 (US)**

## Description

This invention relates to a process for the conversion of light hydrocarbons to gasoline rich in tertiary alkyl ethers and isoalkyl ethers.

One of the more important recent developments in petroleum refining is the establishment of processes to produce high octane gasolines blended with lower aliphatic alkyl ethers as octane boosters and supplementary fuels. $C_5$-$C_7$ methyl alkyl ethers, especially methyl tertiary butyl ether (MTBE) and tertiary amyl methyl ether (TAME) have been found particularly useful for enhancing gasoline octane. Therefore, improvements in the processes related to the production of these and similar ethers in the course of gasoline production are matters of high importance and substantial challenge to research workers in this field.

It is known that alkyl tert-alkyl ethers can be prepared by reacting a primary alcohol with an olefin having a double bond on a tertiary carbon atom. Thus methanol reacts with isobutylene and isopentenes (2 methyl 1-butene or 2 methyl 2-butene) to form respectively methyl tert-butyl ether (MTBE) and methyl tert-amyl ether (TAME). The reaction is selective for tertiary olefins so that it constitutes an attractive process for their removal from olefinic streams in which they are contained together with linear unreactive olefins. The reaction has an equilibrium which is favorable to the synthesis of the ether as the reaction temperature is lowered, in accordance with the negative enthalpy of the reaction. It is known that the reaction is catalyzed by Lewis acids (aluminum trichloride, boron trifluoride), mineral acids (sulfuric acid) and organic acids (alkyl and aryl sulfonic acids, ion exchange resins). Particularly suitable for the task are ion exchange resins in their acid form, such as macroreticular resins of the "Amberlyst 15" type. By means of such last named catalysts it is possible to reach thermodynamic equilibrium within industrially acceptable contact times in the temperature range of 50°-60°C.

U.S. Pat No. 4,262,145 discloses the catalytic reaction of a branched olefin such as isobutylene, 2-methylpentene-2, 2-methylbutene-2, and 2,3-dimethyloctene-2 with a lower alkanol such as methanol to form a mixed ether such as methyl tert-butyl ether. The catalyst disclosed is silicotungstic acid.

A preferred feedstock for the manufacture of MTBE and TAME in petroleum refinery operations is the light hydrocarbon stream from FCC operations. These streams are rich in $C_4$+ tertiary olefins such as isobutylene. However, they also contain significant amounts of linear olefins plus linear and branched paraffins. The linear olefins, particularly propylene and n-butene, are not etherified in the prior art MTBE processes. Conventionally, these linear unreacted olefins are carried through the process and separated downstream. In this regard they represent a burden on the volumetric effectiveness of the etherification process, providing no assistance to the production of ether-rich high octane gasoline.

The present invention resides in a process for the production of ether-rich gasoline, comprising;

(a) contacting an alkanol with a hydrocarbon feedstock containing linear olefins and $C_4$+ tertiary olefins in the presence of an acidic etherification catalyst in a first etherification zone to etherify said tertiary olefins and produce an etherification effluent stream containing alkyl tertiary alkyl ethers;

(b) separating said etherification effluent stream to provide a first stream comprising ether-rich $C_5$+ gasoline and a second stream comprising unreacted alkanol and linear olefins of $C_5$- hydrocarbons;

(c) contacting said second stream with an acidic catalyst in a second etherification zone to etherify said linear olefinic hydrocarbons;

(d) recovering a third stream comprising $C_5$+ gasoline containing alkyl ethers of said $C_5$- linear olefins and a fourth stream comprising unreacted olefins of $C_4$- hydrocarbons from said second etherification zone;

(e) passing said fourth stream to an olefins to higher molecular weight hydrocarbons conversion zone in contact with a zeolite catalyst to produce $C_5$+ gasoline boiling range hydrocarbons and $C_5$- paraffinic hydrocarbon by-product.

The process of the invention utilizes the differing reactivity of tertiary olefins, compared to linear olefins, with alcohols in the catalytic production of ethers. Moreover, unreacted olefins from the etherification reactions are converted to useful higher molecular weight products including $C_5$+ gasoline boiling range hydrocarbons. Further, unreacted paraffins can be dehydrogenated to produce $C_3$-$C_4$ olefins which can be recycled to the etherification process.

More specifically in the process of the invention, a lower alkanol such as methanol, ethanol or isopropanol is reacted with hydrocarbon feedstock containing mixed olefins in a serially integrated process to etherify both branched and linear olefins and produce high octane gasoline containing lower alkyl ethers of branched and linear olefins. In particular tertiary olefins, such as iso-butylene and isoamylene, are converted to tertiary alkyl ethers, particularly methyl tertiary butyl ether (MTBE) and methyl tertiary amyl ether (TAME), while $C_3$-$C_4$ linear olefins are converted to lower alkyl isopropyl ether and sec-butyl ether. Unreacted olefins and by-products from linear olefins etherification such as dimethyl ether and methanol are converted to higher molecular hydrocarbons boiling in the gasoline range.

In the first stage of the process of the invention, a lower alkanol is contacted with a hydrocarbon feedstock containing linear olefins and $C_4+$ tertiary olefins in the presence of an acidic etherification catalyst in a first etherification zone to etherify the tertiary olefins and produce an etherification effluent stream containing alkyl tertiary alkyl ethers.

The lower alcohol fed to the first etherification stage is any primary or secondary alcohol having up to 4 carbon atoms, including the primary alcohols methanol, ethanol, n-propanol, n-butanol and isobutanol; and the secondary alcohols isopropanol and sec-butanol. Methanol is particularly preferred. The lower alcohol is generally present in an excess amount between 1 wt.% to 100 wt%, based upon tertiary olefins converted.

The mixed olefin feed to the first etherification stage preferably comprises $C_4$-$C_7$ isoolefins and $C_3$ to $C_{15}$ linear olefins obeying the chemical formula:

$$R_1-CH=CH-R_2$$

wherein $R_1$ and $R_2$ individually are hydrogen or alkyl groups and the total carbon atoms in $R_1$ plus $R_2$ is from 1 to 13. In a preferred embodiment of the present invention, the linear olefins have 3 to 5 carbon atoms, i.e. the total carbon atoms in $R_1$ plus $R_2$ is 1 to 3. Particularly preferred feeds are propylene, 1-butene and 2-butene.

Typical hydrocarbon feedstock materials for the first etherification reaction include olefinic streams, such as FCC light naphtha and butenes rich in isoolefins. These aliphatic streams are produced in petroleum refineries by catalytic cracking of gas oil and the like.

The first etherification reaction is effected in the presence of an acid etherification catalyst, preferably an ion exchange resin in hydrogen form. Typically, the ion exchange resin is a sulfonic acid ion exchange resin and most preferably is of the Amberlyst 15 type. Other acidic catalysts, such as phosphoric acid modified kieselguhr, silica alumina and acid zeolites, may be employed with varying degrees of success.

The first etherification reaction is conducted at a relatively low temperature (37-93°C) in order to efficiently convert tertiary olefins to high octane alkyl tertiary-alkyl ethers. The first etherification stage preferably consists of a single fixed bed reactor in which the extent of reaction is at least 65% of equilibrium.

The composition of the first etherification effluent comprises unreacted alkanol, hydrocarbons including a major portion of $C_4+$ hydrocarbons containing unreacted linear olefins and lower alkyl tertiary alkyl ethers such as methyl tertiary alkyl ethers. Following the first etherification reaction, the etherification reaction effluent stream is separated into a first $C_5+$ gasoline stream rich in tertiary alkyl ethers and a second hydrocarbon stream containing unreacted lower alkanol and linear olefins for further etherification in a second etherification reaction.

The second etherification reaction converts any unconverted tertiary olefins, excess alcohol, and linear C5- olefins to alkyl tertiary alkyl ethers and alkyl iso-alkyl ethers. The catalyst employed in the second stage is preferably a zeolite and most preferably zeolite Beta. The reaction conditions are as shown in Table 1 below:

## Table 1

### Reaction Conditions

| | Mol Ratio alcohol/olefin | Temp. °C | Press. atm. | WHSV Hr$^{-1}$ |
|---|---|---|---|---|
| Broad | 0.1-10 | 50-300 | 1.0-300 | 0.05-50 |
| Preferred | 0.3-3 | 80-250 | 5-200 | 0.2-20 |
| Most Preferred | 0.5-2 | 100-210 | 10-100 | 0.5-10 |

The principal ether product or products produced depends on the linear olefin and the alcohol charged. In the case of methanol and propylene, for example, the principal reaction product is methyl isopropyl ether. With butene-1 or the cis- or trans-butene-2, methyl sec-butyl ether is formed. In brief, the ethers formed are those predicted by the Markovnikov rule for addition to the double bond of the linear olefin. In the case of the higher molecular weight linear monoolefins, or mixtures of olefins, the principal reaction product is a mixture of such ethers.

The principal by-product formed in the etherification of linear olefins is the ether and water resulting from the autocondensation of the alcohol charged. Other by-products include alcohol resulting from the hydration of the linear monoolefin, and the ether formed by the self-condensation of the latter alcohol. Also formed is a small amount of hydrocarbon believed to be the oligomer of the olefin charged. This hydrocarbon by-product appears to account for less than 5 wt % of the total olefin converted under moderate temperatures, such as at a temperature not higher than about 160°C.

The effluent from the second etherification stage is separated into a third stream comprising $C_5$+ gasoline containing alkyl ethers of the linear olefins and a fourth stream comprising unreacted olefins.

The unreacted olefins from the second etherification reactin are converted, normally by oligomerization, to higher molecular weight hydrocarbons such as gasoline or gasoline and distillate. Oligomerization is preferably effected at a temperature of 215-535 °C, a pressure of 50-2000kPa and a weight hourly space velocity (WHSV) of 0.5-10. Suitable catalysts for the oligomerization step include zeolites having a silica to alumina ratio of 20:1 or greater, a constraint index of 1-12, and an acid cracking activity (alpha value) of 2-200, preferably 2-35. Representative zeolites are ZSM-5 (U.S. Patent No. 3,702,886 and U.S. Patent No. Reissue 29,948), ZSM-11 (U.S. Patent No. 3,709,979), ZSM-12 (U.S. Patent No. 3,832,449), ZSM-23 (U.S. Patent No. 4,076,842), ZSM-35 (U.S. Patent No. 4,016,245) and ZSM-48 (U.S. Patent No. 4,375,573), with ZSM-5 being particularly preferred.

Preferably, the unreacted paraffins in the effluent of the oligomerization process are passed to a dehydrogenation zone where they are converted to olefins. The $C_4$ olefin fraction from dehydrogenation is then recycled to the first etherification zone for conversion to ethers.

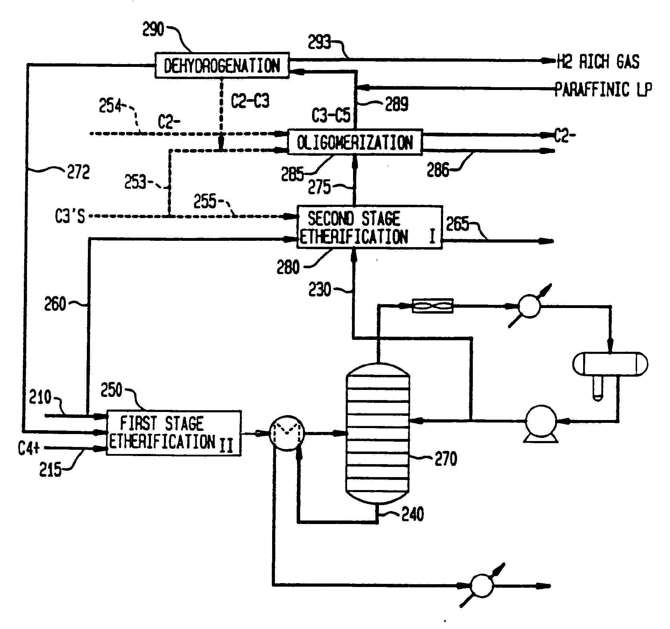

The invention will now be more particularly described with reference to the accompanying drawing, which is a schematic flow diagram of a process according to a preferred embodiment of the present invention.

Referring to the drawing, in the process shown methanol and hydrocarbon reactants are passed to a first etherification reactor 250 in conduits 210 and 215. The etherification product is passed as an effluent stream to a separator 270, where methanol is removed as an overhead stream, preferably as an azeotropic mixture with $C_5$- paraffinic and olefinic hydrocarbons, which is then passed by conduit 230 to a second etherification zone 280. A bottom fraction is withdrawn from separator 270 through conduit 240 and contains methyl tertiary alkyl ethers, such as MTBE and TAME, in admixture with $C_5$+ gasoline. The gasoline separated exhibits a high motor octane value and high research octane value.

In the second etherification zone 280 linear olefins are converted to methyl ethers, optionally with added methanol from conduit 260 and $C_3$ hydrocarbons from conduit 255. Product $C_5$+ gasoline rich in ethers is separated through conduit 265 while byproducts, including dimethyl ether (DME) and methanol, and unconverted $C_4$- hydrocarbons are passed through conduit 275 to oligomerization unit 285. Optionally, FCC $C_3$'s and ethene are introduced into the unit 285 as feedstock through, respectively, conduits 253 and 254. In the unit 285 olefins are converted to $C_5$+ gasoline, which is recovered through conduit 286. Unreacted $C_3$-$C_5$ paraffins separated from the unit 285 are passed via conduit 289 to dehydrogenation unit 290, optionally with added LPG paraffinic feed. $C_4$ olefins are separated from the dehydrogenation reactor effluent and passed through conduit 292 to the first etherification stage for further etherification in conjunction with fresh hydrocarbon feedstock. By-product hydrogen-rich gas is recovered from the dehydrogenation unit 290 through conduit 293.

## Claims

1. A process for the production of ether-rich gasoline, comprising;

   (a) contacting an alkanol with a hydrocarbon feedstock containing linear olefins and $C_4$+ tertiary olefins in the presence of an acidic etherification catalyst in a first etherification zone to etherify said tertiary olefins and produce an etherification effluent stream containing alkyl tertiary alkyl ethers;

   (b) separating said etherification effluent stream to provide a first stream comprising ether-rich $C_5$+ gasoline and a second stream comprising unreacted alkanol and linear olefins of $C_5$- hydrocarbons;

   (c) contacting said second stream with an acidic catalyst in a second etherification zone to etherify said linear olefinic hydrocarbons;

   (d) recovering a third stream comprising $C_5$+ gasoline containing alkyl ethers of said $C_5$- linear olefins and a fourth stream comprising unreacted olefins of $C_4$- hydrocarbons from said second etherification zone;

   (e) passing said fourth stream to an olefins to higher molecular weight hydrocarbons conversion zone in contact with a zeolite catalyst to produce $C_5$+ gasoline boiling range hydrocarbons and $C_5$- paraffinic hydrocarbon by-product.

2. The process of claim 1 including the step of introducing a fresh stream of said alkanol to the second etherification zone.

3. The process of claim 1 or claim 2 wherein step (a) is effected with an excess of said alkanol over $C_4$+

4

tertiary olefins.

4. The process of claim 3 wherein said excess of said alkanol is 1 to 100% by weight.

5. The process of any preceding claim wherein step (a) is effected at a temperature of 37 to 93°C in the presence of ion exchange resin catalyst.

6. The process of any preceding claim wherein step (c) is effected at a temperature of 50 to 300°C in the presence of a zeolite catalyst.

7. The process of claim 6 wherein the zeolite catalyst is zeolite beta.

8. The process of any preceding claim wherein step (e) is effected in the presence of a zeolite having a Constraint Index of 1 to 12.

9. The process of claim 8 wherein the zeolite is ZSM-5.

10. The process of any preceding claim including the further steps of:
(f) passing the $C_5$- paraffinic hydrocarbon by-product from step (e) to a dehydrogenation zone to convert said by-product to olefins and hydrogen rich fuel gas; and
(g) recycling the olefins from step (f) to the first etherification zone.


**Patentansprüche**

1. Verfahren zur Herstellung von etherreichem Benzin, das umfaßt:
(a) In Kontakt bringen eines Alkanols mit einem Kohlenwasserstoff-Einsatzmaterial, das lineare Olefine und tertiäre $C_4$-Olefine enthält, in Gegenwart eines sauren Veretherungskatalysators in einer ersten Veretherungszone, um das tertiäre Olefin zu verethern und einen Veretherung-Abstrom herzustellen, der tertiäre Alkylether enthält;
(b) Auftrennen des Veretherung-Abstroms, um einen ersten Abstrom aus etherreichem $C_5$-Benzin und einen zweiten Abstrom aus nicht umgesetztem Alkanol und linearen Olefinen der $C_5$-Kohlenwasserstoffe zu schaffen;
(c) In Kontakt bringen des zweiten Stroms mit einem sauren Katalysator in einer zweiten Veretherungszone, um die linearen Olefinkohlenwasserstoffe zu verethern;
(d) Gewinnen eines dritten Stroms, der $C_5$-Benzin mit Alkylether der linearen $C_5$-Olefine umfaßt, und eines vierten Stroms, der nicht umgesetzte Olefine der $C_4$-Kohlenwasserstoffe umfaßt, aus der zweiten Veretherungszone;
(e) Zuführen des vierten Stroms zu einer Umwandlungszone für Olefine zu höhermolekularen Kohlenwasserstoffen in Kontakt mit einem Zeolith-Katalysator, um Kohlenwasserstoffe im Siedebereich von $C_5$-Benzin und $C_5$-Paraffinkohlenwasserstoff-Nebenprodukt herzustellen.

2. Verfahren nach Anspruch 1, das den Schritt der Einleitung eines frischen Stroms des Alkanols zur zweiten Veretherungszone einschließt.

3. Verfahren nach Anspruch 1 oder 2, worin Schritt (a) mit einen Überschuß des Alkanols gegenüber den tertiären $C_4$-Olefinen ausgeführt wird.

4. Verfahren nach Anspruch 3, worin der Überschuß des Alkanols 1 bis 100 Gew.% beträgt.

5. Verfahren nach einem der vorangegangenen Ansprüche, worin Schritt (a) bei einer Temperatur von 37 bis 93°C in Gegenwart eines Ionenaustauschharz-Katalysators ausgeführt wird.

6. Verfahren nach einem der vorangegangenen Ansprüche, worin Schritt (c) bei einer Temperatur von 50 bis 200°C in Gegenwart eines Zeolithkatalysators ausgeführt wird.

7. Verfahren nach Anspruch 6, worin der Zeolithkatalysator Zeolith-beta ist.

8. Verfahren nach einem der vorangegangenen Ansprüche, worin Schritt (e) in Gegenwart eines Zeoliths mit einem Beanspruchungsindex von 1 bis 12 ausgeführt wird.

9. Verfahren nach Anspruch 8, worin der Zeolith ZSM-5 ist.

10. Verfahren nach einem der vorangegangenen Ansprüche, daß die weiteren Schritte einschließt:
(f) Zuführen des $C_5$-Paraffinkohlenwasserstoff-Nebenproduktes aus Schritt (e) zu einer Dehydrierungszone, um das Nebenprodukt in Olefine und kohlenwasserstoffreiches Brenngas überzuführen; und
(g) Rückführen der Olefine aus Schritt (f) zur ersten Veretherungszone.

## Revendications

1. Un procédé de production d'une essence riche en éther comprenant:
(a) la mise en contact d'un alcanol avec une charge hydrocarbonée contenant des oléfines linéaires et des oléfines tertiaires en $C_4^+$ en présence d'un catalyseur d'éthérification acide dans une première zone d'éthérification pour éthérifier lesdites oléfines tertiaires et produire un courant d'effluent d'éthérification contenant des alkyl-t-alkyléthers;
(b) la séparation dudit courant d'effluent d'éthérification pour former un premier courant comprenant une essence en $C_5^+$ riche en éther et un second courant comprenant l'alcanol n'ayant pas réagi et les oléfines linéaires d'hydrocarbures en $C_5^-$;
(c) la mise en contact dudit second courant avec un catalyseur acide dans une seconde zone d'éthérification pour éthérifier lesdits hydrocarbures oléfines linéaires;
(d) la récupération d'un troisième courant comprenant de l'essence en $C_5^+$ contenant des alkyléthers, lesdites oléfines linéaires en $C_5^-$ et un quatrième courant comprenant les oléfines n'ayant pas réagi d'hydrocarbures en $C_4^-$ provenant de ladite seconde zone d'éthérification;
(e) passage dudit quatrième courant dans une zone de conversion d'oléfines en hydrocarbures de poids moléculaire plus élevé au contact d'un catalyseur zéolitique pour produire des hydrocarbures en $C_5^-$ bouillant dans l'intervalle des essences et des hydrocarbures paraffiniques en $C_5^-$ en tant que sous-produits.

2. Le procédé selon la revendication 1, incluant l'étape d'introduction d'un courant frais dudit alcanol dans la seconde zone d'éthérification.

3. Le procédé selon la revendication 1 ou 2, caractérisé en ce l'étape (a) est réalisée avec un excès dudit alcanol par rapport à des oléfines tertiaires en $C_4^+$.

4. Le procédé selon la revendication 3, caractérisé en ce ledit excès dudit alcanol est 1 à 100% en poids.

5. Le procédé selon l'une quelconque des revendications précédentes, caractérisé en ce l'étape (a) est mise en oeuvre à une température de 37°C à 93°C en présence d'un catalyseur à base d'une résine échangeuse d'ions.

6. Le procédé selon l'une quelconque des revendications précédentes, caractérisé en ce l'étape (c) est effectuée à une température de 50°C à 300°C en présence d'un catalyseur zéolitique.

7. Le procédé selon la revendication 6, caractérisé en ce que le catalyseur correspond à la zéolite béta.

8. Le procédé selon l'une quelconque des revendications précédentes, caractérisé en ce l'étape (e) est effectuée en présence d'une zéolite présentant un indice de contrainte de 1 à 12.

9. Le procédé selon la revendication 8, caractérisé en ce que la zéolite est la ZSM-5.

10. Le procédé selon l'une quelconque des revendications précédentes, incluant les étapes supplémentaires de:
(f) passage du sous-produit hydrocarboné paraffinique en $C_5^-$ de l'étape (e) dans une zone de déshydrogénation pour convertir ledit sous-produit en un gaz combustible riche en hydrogène et en oléfine; et
(g) recyclage des oléfines provenant de l'étape (f) vers la première zone d'éthérification.

FIG. 1